# EUROPÄISCHE PATENTSCHRIFT

(11) **EP 4 556 045 B1**
(45) Veröffentlichungstag und Bekanntmachung des Hinweises auf die Patenterteilung: **22.07.2026**
(21) Anmeldenummer: 24211978.2
(22) Anmeldetag: 11.11.2024
(51) Int. Cl.: A61M 25/00, A61M 25/04, A61M 25/06, A61N 1/05, A61N 1/36

(54) **KATHETER ZUR VERABREICHUNG EINES LOKALANÄSTHETIKUMS**
CATHETER FOR DELIVERY OF LOCAL ANESTHETIC
CATHÉTER POUR L'ADMINISTRATION D'UN ANESTHÉSIQUE LOCAL

(30) Priorität: 14.11.2023 DE 102023131697
(43) Veröffentlichungstag der Anmeldung: 21.05.2025
(73) Patentinhaber: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(56) Entgegenhaltungen:
- EP-B1- 3 274 036
- US-A- 5 968 012

## Beschreibung

Die Erfindung betrifft einen Katheter zur Verabreichung eines Lokalanästhetikums, aufweisend einen Katheterschaft, der zwischen einem proximalen Ende und einem distalen Ende längserstreckt ist, eine Katheterspitze, die an dem distalen Ende angeordnet ist, und aufweisend einen Katheteransatz, der an dem proximalen Ende angeordnet ist.

Derartige Katheter sind im Bereich der Medizintechnik allgemein bekannt und zur Verwendung bei einer Schmerztherapie vorgesehen. Bei einer als peripherer Nervenblock (PNB) bezeichneten Schmerztherapie wird der Katheter unter Zuhilfenahme einer Einführhilfe so weit distal vorgeschoben, bis die Katheterspitze unmittelbar an dem zu betäubenden Nerv platziert ist. Als Einführhilfen werden Kanülen und/oder Kapillare verwendet, wobei prinzipiell zwischen unterschiedlichen Anlagetechniken unterschieden werden kann ("Kanüle über die Nadel", "Kanüle durch die Nadel", "Kanüle durch das Kapillar"). Zur Betäubung des Nervs wird das Lokalanästhetikum über einen oder mehrere im Bereich der Katheterspitze angeordnete Katheterauslässe abgegeben.

Aus der EP 3 274 036 B1 ist ein Katheter mit den oberbegrifflichen Merkmalen des Anspruchs 1 bekannt. Der bekannte Katheter ist zum Verabreichen von Anästhetika an eine periphere Nervenstelle vorgesehen. Der Katheter weist einen Katheterschlauch und einen aufblasbaren Ballon auf, der an einem distalen Ende des Katheterschlauchs angeordnet ist und der Verankerung des Katheterschlauchs dient.

Aus der US 5 968 012 A ist ein Ballonkatheter mit einem aufblasbaren Ballon und einem Längenkompensator bekannt, der als Akkordeonabschnitt gestaltet ist. Beim Aufblasen des Ballons wird der Akkordeonabschnitt gedehnt, um die Längenänderung des Ballons zu kompensieren.

Aufgabe der Erfindung ist es, einen Katheter der eingangs genannten Art bereitzustellen, der eine verbesserte Schmerztherapie ermöglicht.

Diese Aufgabe wird dadurch gelöst, dass der Katheterschaft einen Streckabschnitt aufweist, der derart leicht streckbar ausgebildet ist, dass bei einer proximalen Zugbelastung des Katheteransatzes eine wenigstens abschnittsweise Streckung des Katheterschafts erfolgt, wodurch eine proximale Dislokation der Katheterspitze vermieden ist. Die Erfindung geht von der Erkenntnis aus, dass ein unbeabsichtigtes Zurückziehen der Katheterspitze (proximale Dislokation) zu einer nicht ausreichenden Umspülung des Nervs mit dem Lokalanästhetikum führen und dadurch die Wirksamkeit der Schmerztherapie beeinträchtigen kann. Durch die erfindungsgemäße Lösung werden solche Dislokationen der Katheterspitze vermieden. Zu diesem Zweck weist der Katheterschaft den besagten Streckabschnitt auf. Der Streckabschnitt ist leicht streckbar ausgebildet. Leicht streckbar meint, dass der Streckabschnitt eine vergleichsweise geringe Zugfestigkeit und/oder eine vergleichsweise hohe Zugnachgiebigkeit aufweist und folglich leicht streckbar, d.h. in seiner Länge dehnbar, ist. Bei einer proximalen Zugbelastung des Katheteransatzes und damit des Katheterschafts, die beispielsweise durch eine Mobilisierung des Patienten verursacht sein kann, wird der Streckabschnitt gestreckt, d.h. gelängt und/oder gedehnt. Durch diese Längenzunahme des Katheterschafts im Bereich des Streckabschnitts erfolgt eine entsprechend verringerte und im besten Fall keine proximale Dislokation der Katheterspitze. Bei einer Ausgestaltung ist der Streckabschnitt zur elastischen Streckung ausgebildet. Bei einer weiteren Ausgestaltung ist der Streckabschnitt zur plastischen Streckung ausgebildet. Bei einer weiteren Ausgestaltung ist der Streckabschnitt zur elastischplastischen Streckung ausgebildet. Bei einer Ausgestaltung wird die vergleichsweise leichte Streckbarkeit des Streckabschnitts durch eine entsprechende Materialwahl für den Streckabschnitt erreicht. Bei einer weiteren Ausgestaltung weist der Streckabschnitt alternativ oder zusätzlich eine leicht streckbare Gestaltgebung auf, beispielsweise eine ziehharmonikaförmige oder spiralförmige Gestalt. Vorzugsweise ist der Katheteransatz zur lösbaren Verbindung mit einer Einführhilfe eingerichtet, wobei die Einführhilfe insbesondere als Kapillar oder als Kanüle gestaltet sein kann.

In Ausgestaltung der Erfindung weist der Streckabschnitt eine Zugnachgiebigkeit von wenigstens 10 mm/N, bevorzugt von wenigstens 100 mm/N, besonders bevorzugt von wenigstens 1000 mm/N, auf. Die vorgenannten Wertebereiche für die Zugnachgiebigkeit des Streckabschnitts haben sich als besonders vorteilhaft erwiesen, um die besagte proximale Dislokation der Katheterspitze zu vermeiden.

In weiterer Ausgestaltung der Erfindung weist der Streckabschnitt eine leicht streckbare Gestaltgebung auf. Bei dieser Ausgestaltung der Erfindung wird die leichte Streckbarkeit des Streckabschnitts nicht unbedingt durch eine geeignete Materialwahl, sondern durch eine entsprechende Gestaltgebung erreicht. Die Längenzunahme erfolgt nicht unbedingt infolge einer elastischen und/oder plastischen Deformation, sondern aufgrund einer sonstigen Gestaltänderung des Streckabschnitts, beispielsweise aufgrund einer Ziehharmonikabewegung. Grundsätzlich kann der Streckabschnitt jede zum Zwecke der leichten Streckbarkeit geeignete Gestaltgebung aufweisen. Bei dieser Ausgestaltung der Erfindung weist der Katheterschaft folglich wenigstens im Bereich des Streckabschnitts die besagte leicht streckbare Gestaltgebung auf, wobei der Katheterschaft abseits des Streckabschnitts eine unterschiedliche und weniger leicht streckbare Gestaltgebung aufweist, vorzugsweise eine aus dem Stand der Technik bekannte glattwandig zylindrische Gestaltung. Sofern der Streckabschnitt die gesamte Länge des Katheterschafts einnimmt, weist folglich der gesamte Katheterschaft die besagte leicht streckbare Gestaltgebung auf.

In weiterer Ausgestaltung der Erfindung ist die leicht streckbare Gestaltgebung eine ziehharmonikaartige Gestaltgebung. Bei dieser Ausgestaltung ist der Streckabschnitt nach Art einer Ziehharmonika gestaltet. Hierdurch kann der Streckabschnitt bei einer entsprechenden Zugbelastung leicht gestreckt werden. Die ziehharmonikaartige Gestaltgebung kann insbesondere durch eine gefaltete oder wellenförmige Ausbildung des Streckabschnitts erreicht werden, ähnlich wie bei einem abknickbaren Trinkhalm und/oder einem längselastischen Gartenschlauch. Denkbar und möglich ist zudem eine wendelförmige Gestaltung des Streckabschnitts.

In weiterer Ausgestaltung der Erfindung ist der Streckabschnitt aus einem leicht streckbaren Material gefertigt. Vorzugsweise ist das leicht streckbare Material ein Kunststoffmaterial. Bei dieser Ausgestaltung der Erfindung ist der Katheterschaft folglich wenigstens im Bereich des Streckabschnitts aus dem besagten leicht streckbaren Material gefertigt, wobei der Katheterschaft abseits des Streckabschnitts aus einem weniger leicht streckbaren Material gefertigt ist. Sofern der Streckabschnitt die gesamte Länge des Katheterschafts einnimmt, ist folglich der gesamte Katheterschaft aus dem besagten leicht streckbaren Material gefertigt.

In weiterer Ausgestaltung der Erfindung weist das leicht streckbare Material Polyethylen und/oder Silikon auf. Alternativ besteht das leicht streckbare Material aus Polyethylen und/oder Silikon. Die genannten Materialien haben sich als besonders vorteilhaft für die vorliegende Anwendung erwiesen.

In weiterer Ausgestaltung der Erfindung ist der Streckabschnitt über eine gesamte Länge des Katheterschafts längserstreckt. Folglich ist bei dieser Ausgestaltung der Erfindung der gesamte Katheterschaft leicht streckbar. Diese Ausgestaltung der Erfindung erlaubt eine vereinfachte Herstellung, da der Katheterschaft aus ein- und demselben Material gefertigt und/oder ein- und dieselbe Gestaltgebung aufweisen kann. Dies im Unterschied zu Ausführungsformen, bei denen der Streckabschnitt lediglich einen Teil der Gesamtlänge des Katheterschafts einnimmt.

In weiterer Ausgestaltung der Erfindung ist der Streckabschnitt lediglich über einen Teil der Gesamtlänge des Katheterschafts längserstreckt und der Katheterschaft weist abseits des Streckabschnitts eine Zugnachgiebigkeit auf, die maximal 20 %, bevorzugt maximal 5 %, besonders bevorzugt maximal 2 %, der Zugnachgiebigkeit des Streckabschnitts beträgt. Mit anderen Worten ausgedrückt, weist der Katheterschaft abseits des Streckabschnitts eine Zugfestigkeit auf, die wenigstens fünfmal, bevorzugt wenigstens zwanzigmal, besonders bevorzugt wenigstens fünfzigmal, höher ist als die Zugfestigkeit des Streckabschnitts. Bei dieser Ausgestaltung der Erfindung ist der Streckabschnitt vorzugsweise im Bereich des distalen Endes des Katheterschafts angeordnet.

In weiterer Ausgestaltung der Erfindung weist der Katheterschaft abseits des Streckabschnitts eine unterschiedliche Gestaltgebung auf und/oder ist aus einem unterschiedlichen Material gefertigt. Die unterschiedliche Gestaltgebung ist weniger leicht streckbar und vorzugsweise glattwandig zylindrisch. Das unterschiedliche Material ist weniger leicht streckbar und vorzugsweise ein für Katheterschäfte/Katheterschläuche übliches, dem Fachmann bekanntes Kunststoffmaterial.

In weiterer Ausgestaltung der Erfindung ist eine Streckungsbegrenzungseinrichtung vorhanden und zur Begrenzung der Streckung des Streckabschnitts eingerichtet. Durch die Streckungsbegrenzungseinrichtung kann eine über ein zulässiges Maß hinausgehende Streckung des Streckabschnitts begrenzt werden. Die Begrenzung der Streckung des Streckabschnitts wirkt einer mechanischen Überbeanspruchung des Streckabschnitts entgegen und verhindert damit ein Abreißen des Katheterschafts. Die Streckungsbegrenzungseinrichtung ist bei unterschiedlichen Ausgestaltungen unterschiedlich ausgeführt und kann grundsätzlich jede für den vorliegenden Zweck geeignete Form und Funktion aufweisen.

In weiterer Ausgestaltung der Erfindung weist die Streckungsbegrenzungseinrichtung ein längserstrecktes Zugelement auf, das den Streckabschnitt unter Ausbildung einer Zugentlastung überbrückt. Das längserstreckte Zugelement bewirkt eine mechanische Überbrückung des Streckabschnitts, sobald dieser eine maximal zulässige Streckung erreicht. Dabei bildet das Zugelement die besagte Zugentlastung aus. Das längserstreckte Zugelement kann auf jede für den vorliegenden Zweck geeignete Weise gestaltet sein.

In weiterer Ausgestaltung der Erfindung weist das längserstreckte Zugelement einen elektrisch leitfähigen Draht auf, der zur Übertragung von Stromimpulsen zur Neurostimulation eingerichtet ist. Der Draht weist folglich eine vorteilhafte Mehrfachfunktion auf. Zum einen fungiert der Draht als Zugentlastung. Zum anderen fungiert der Draht zusätzlich als Leiterdraht für die besagte Neurostimulation. Bei dieser Ausgestaltung der Erfindung weist der Katheterschaft und/oder die Katheterspitze wenigstens eine Stimulationselektrode auf, die mittels des leitfähigen Drahts elektrisch leitfähig kontaktiert oder durch den Draht gebildet ist.

In weiterer Ausgestaltung der Erfindung weist die Katheterspitze eine Fixiereinrichtung auf, die zum Fixieren der Katheterspitze in proximaler Richtung eingerichtet ist, um eine proximale Dislokation der Katheterspitze zu erschweren. Die Fixiereinrichtung wirkt der besagten proximalen Dislokation zusätzlich entgegen. Mit anderen Worten wird die Katheterspitze in angelegtem Zustand des Katheters mittels der Fixiereinrichtung im Bereich des zu betäubenden Nervs fixiert. Die Fixiereinrichtung kann jede für den vorliegenden Zweck geeignete Form und Funktion aufweisen. Beispielsweise kann die Katheterspitze eine spezifische Gestaltgebung aufweisen, welche die Fixiereinrichtung ausbildet. Alternativ oder zusätzlich kann die Katheterspitze mit Fixierelementen versehen sein, die einer proximalen Bewegung der Katheterspitze entgegenwirken. Zu diesem Zweck können die besagten Fixierelemente zum reibschlüssigen und/oder formschlüssigen Zusammenwirken mit einem die Katheterspitze umgebenden Körpergewebe eingerichtet sein.

Die Erfindung betrifft zudem eine Katheteranordnung mit einem Katheter gemäß der vorhergehenden Beschreibung und mit einer Einführhilfe, die zwischen einem distalen Ende und einem proximalen Ende längserstreckt ist und ein Lumen zum Einführen des Katheterschafts sowie an dem proximalen Ende der Einführhilfe angeordneten Konnektor zum Ausbilden einer lösbaren Verbindung mit dem Katheteransatz aufweist. Die Einführhilfe kann auch als Kapillar bezeichnet werden. Der Katheteransatz ist zur lösbaren Verbindung mit dem Konnektor der Einführhilfe eingerichtet und weist vorzugsweise einen komplementären Konnektor auf. Für den vorliegenden Zweck geeignete Konnektoren, beispielsweise Luer-Konnektoren, sind dem Fachmann bekannt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Seitenansicht eine Ausführungsform eines erfindungsgemäßen Katheters mit einem Katheterschaft, der einen Streckabschnitt aufweist,
- Fig. 2: eine weitere schematische Seitenansicht des Katheters nach Fig. 1, wobei der Streckabschnitt einen gestreckten Zustand einnimmt,
- Fig. 3, 4: eine schematische Seitenansicht einer Ausführungsform eines Streckabschnitts mit leicht streckbarer Gestaltgebung in einem nicht gestreckten Zustand (Fig. 3) und in einem gestreckten Zustand (Fig. 4),
- Fig. 5: eine schematische Seitenansicht einer Katheteranordnung mit einem Katheter nach den Fig. 1 und 2 und einer Einführhilfe, wobei die Einführhilfe in einem Längsschnitt dargestellt ist,
- Fig. 6: eine weitere Ausführungsform eines erfindungsgemäßen Katheters in schematischer Seitenansicht, wobei der Streckabschnitt eine gesamte Länge des Katheterschafts einnimmt,
- Fig. 7: eine weitere Ausführungsform eines erfindungsgemäßen Katheters in schematischer Seitenansicht und mit einer Streckungsbegrenzungseinrichtung zur Begrenzung der Streckung des Streckabschnitts,
- Fig. 8: eine weitere Ausführungsform eines erfindungsgemäßen Katheters in schematischer Seitenansicht, wobei die Streckungsbegrenzungseinrichtung einen Draht aufweist, und
- Fig. 9: eine weitere Ausführungsform eines erfindungsgemäßen Katheters in schematischer Seitenansicht und mit einer Fixiereinrichtung.

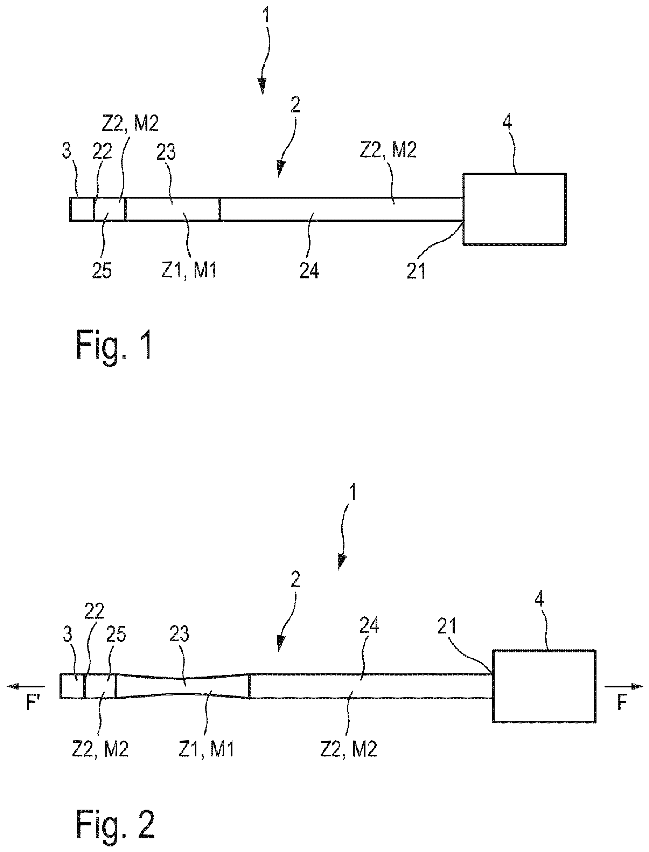

Gemäß Fig. 1 ist ein Katheter 1 zur Verabreichung eines Lokalanästhetikums vorgesehen. Im Speziellen dient der Katheter 1 als Schmerzkatheter bei einer Schmerztherapie, die auch als peripherer Nervenblock bezeichnet wird.

Der Katheter 1 weist einen Katheterschaft 2, eine Katheterspitze 3 und einen Katheteransatz 4 auf.

Der Katheterschaft 2 ist zwischen einem proximalen Ende 21 und einem distalen Ende 22 längserstreckt.

Die Katheterspitze 3 ist an dem distalen Ende 22 des Katheterschafts 2 angeordnet. Die Katheterspitze 3 ist bei der gezeigten Ausführungsform gesondert von dem Katheterschaft 2 gefertigt und fest mit dessen distalem Ende 22 verbunden. Alternativ kann die Katheterspitze 3 ein integraler Abschnitt des Katheterschafts 2 sein und dessen distales Ende 22 bilden.

Der Katheteransatz 4 ist an dem proximalen Ende 21 des Katheterschafts 2 angeordnet. Der Katheteransatz 4 ist fest mit dem proximalen Ende 21 verbunden. Der Katheteransatz 4 kann auch als Katheter-Hub (englisch: catheter hub) bezeichnet werden.

Es versteht sich, dass der Katheter 1 wenigstens ein durch den Katheterschaft 2 und den Katheteransatz 4 längserstrecktes Lumen und wenigstens einen fluidleitend mit dem Lumen verbundenen Katheterauslass zur Abgabe des Lokalanästhetikums aufweist. Der wenigstens eine Katheterauslass und das Lumen sind vorliegend nicht im Detail gezeigt. Der Katheterauslass oder die Katheterauslässe sind vorzugsweise im Bereich der Katheterspitze angeordnet. Dabei kann der wenigstens eine Katheterauslass axial durch die Katheterspitze und/oder radial durch den Katheterschaft erstreckt sein. Die Anzahl und Anordnung der Katheterauslässe ist im Hinblick auf die vorliegende Erfindung nicht wesentlich, so dass weitere Details nicht erläutert werden.

Zur Verabreichung des Lokalanästhetikums wird der Katheter 1 auf eine dem Fachmann bekannte Weise angelegt und so weit distal vorgeschoben, bis die Katheterspitze 3 in unmittelbarer Nähe zu dem zu betäubenden Nerv platziert ist. Das Anlegen des Katheters 1 erfolgt vorzugsweise unter Zuhilfenahme einer Einführhilfe, was nachfolgend noch näher erläutert wird.

Kommt es während der Schmerztherapie zu einer unbeabsichtigten proximalen Bewegung der Katheterspitze 3, einer sogenannten (proximalen) Dislokation, wird der zu betäubende Nerv nicht mehr ausreichend von dem Lokalanästhetikum umspült. Hierdurch kann die Wirksamkeit der Schmerztherapie beeinträchtigt werden. Um dem entgegenzuwirken, weist der Katheterschaft 2 einen Streckabschnitt 23 auf.

Der Streckabschnitt 23 ist derart leicht streckbar ausgebildet, dass bei einer proximalen Zugbelastung F (siehe Fig. 2) des Katheteransatzes 4 eine wenigstens abschnittsweise Streckung des Katheterschafts 2 erfolgt. Dadurch kann eine proximale Dislokation der Katheterspitze 3 vermieden werden.

Fig. 2 zeigt den Streckabschnitt 23 in einem gestreckten Zustand. Der gestreckte Zustand des Streckabschnitts 23 wird durch die besagte proximale Zugbelastung F (und eine entgegengesetzte distale Reaktionskraft F') hervorgerufen. Zur exemplarischen Verdeutlichung greift die proximale Zugbelastung F an dem Katheteransatz 4 an und die Reaktionskraft F' greift an der Katheterspitze 3 an.

Aufgrund der axialen Belastung des Katheterschafts 2 wird der Streckabschnitt 23 axial gestreckt und/oder gelängt, insbesondere gedehnt. Diese Längenzunahme kann je nach spezifischer Gestaltgebung und Materialwahl elastisch, plastisch oder elastisch-plastisch sein.

Bei der in den Fig. 1 und 2 gezeigten Ausführungsform ist der Streckabschnitt 23 lediglich über einen Teil einer Gesamtlänge des Katheterschafts 2 längserstreckt. Dabei sind die Anordnung des Streckabschnitts 23 und dessen Anteil an der Gesamtlänge als exemplarisch zu verstehen.

Vorliegend ist der Streckabschnitt 23 im Bereich des distalen Endes 22 des Katheterschafts 2 angeordnet. Der Katheterschaft 2 weist weiter einen proximalen Schaftabschnitt 24 und einen distalen Schaftabschnitt 25 auf. Der proximale Schaftabschnitt 24 ist axial zwischen dem proximalen Ende 21 und dem Streckabschnitt 23 angeordnet. Der proximale Schaftabschnitt 24 umfasst vorliegend das proximale Ende 21 des Katheterschafts 2 und ist einends fest mit dem Katheteransatz 4 verbunden. Andernends ist der proximale Schaftabschnitt 24 fest mit dem Streckabschnitt 23 verbunden. Der distale Schaftabschnitt 25 ist axial zwischen der Katheterspitze 3 und dem Streckabschnitt 23 angeordnet. Der distale Schaftabschnitt 25 umfasst vorliegend das distale Schaftende 22 und ist einends fest mit der Katheterspitze 3 verbunden. Andernends ist der distale Schaftabschnitt 25 fest mit dem Streckabschnitt 23 verbunden.

Bei einer in den Figuren nicht gezeigten Ausführungsform ist kein distaler Schaftabschnitt 25 vorhanden und der Streckabschnitt 23 ist stattdessen unmittelbar mit der Katheterspitze 3 verbunden.

Der proximale Schaftabschnitt 24 und der distale Schaftabschnitt 25 sind weniger leicht streckbar als der Streckabschnitt 23. Mit anderen Worten ausgedrückt, weist der Streckabschnitt 23 eine höhere Zugnachgiebigkeit auf als der proximale Schaftabschnitt 24 und der distale Schaftabschnitt 25. Umgekehrt ausgedrückt, weisen der proximale Schaftabschnitt 24 und der distale Schaftabschnitt 25 eine höhere Zugfestigkeit auf als der Streckabschnitt 23. Die unterschiedliche Gestaltung im Hinblick auf die Streckbarkeit kann über eine entsprechend unterschiedliche Materialwahl und/oder Gestaltung erreicht werden.

Bei der in den Fig. 1 und 2 gezeigten Ausführungsform weist der Streckabschnitt 23 eine Zugnachgiebigkeit Z1 auf. Abseits des Streckabschnitts 23, d.h. im Bereich des proximalen Schaftabschnitts 24 und des distalen Schaftabschnitts 25, weist der Katheterschaft 2 eine geringere Zugnachgiebigkeit Z2 auf. Die beiden Zugnachgiebigkeiten Z1, Z2 können auch als erste Zugnachgiebigkeit Z1 und zweite Zugnachgiebigkeit Z2 bezeichnet werden.

Die (erste) Zugnachgiebigkeit Z1 des Streckabschnitts beträgt vorliegend 100 mm/N. Bei in den Figuren nicht gezeigten Ausführungsformen beträgt die Zugnachgiebigkeit Z1 wenigstens 10 mm/N.

Die (zweite) Zugnachgiebigkeit Z2 des Katheterschafts 2 abseits des Streckabschnitts 23 beträgt vorliegend lediglich 2 % der (ersten) Zugnachgiebigkeit Z1. Bei in den Figuren nicht gezeigten Ausführungsformen beträgt die zweite Zugnachgiebigkeit maximal 20 %, bevorzugt maximal 5 %, der ersten Zugnachgiebigkeit Z1.

Bei der Ausführungsform nach den Fig. 1 und 2 ist der Streckabschnitt 23 aus einem Material M1 gefertigt. Abseits des Streckabschnitts 23, d.h. im Bereich des proximalen Schaftabschnitts 24 und des distalen Schaftabschnitts 25, ist der Katheterschaft 2 aus einem unterschiedlichen Material M2 gefertigt. Die beiden Materialien M1, M2 können auch als erstes Material M1 und zweites Material M2 bezeichnet werden.

Bei beiden Materialien M1, M2 handelt es sich vorliegend um für medizinische Anwendungen geeignete Kunststoffwerkstoffe. Die Materialien M1, M2 sind wenigstens im Hinblick auf ihre Zugnachgiebigkeit unterschiedlich.

Vorliegend weist das erste Material M1 Polyethylen und/oder Silikon auf. Bei einer in den Figuren nicht gezeigten Ausführungsform ist das erste Material Polyethylen. Bei einer weiteren in den Figuren nicht gezeigten Ausführungsform ist das erste Material Silikon.

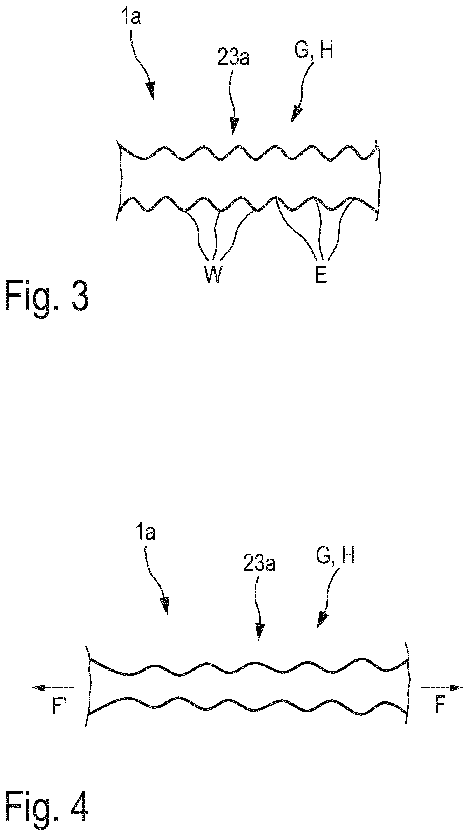

Bei einer Ausführungsform nach den Fig. 1 und 2 wird die leichte Streckbarkeit des Streckabschnitts 23 folglich über eine entsprechende Materialwahl erreicht. Alternativ oder zusätzlich kann der Streckabschnitt eine leicht streckbare Gestaltgebung aufweisen. Eine solche leicht streckbare Gestaltgebung ist exemplarisch in den Fig. 3 und 4 gezeigt.

Die Fig. 3 und 4 zeigen eine Ausführungsform eines Katheters 1a, bei welcher der Streckabschnitt 23a eine leicht streckbare Gestaltgebung G aufweist. Im Speziellen ist der Streckabschnitt 23a nach Art einer Ziehharmonika gestaltet und weist insoweit eine ziehharmonikaartige Gestaltgebung H auf. Der Katheterschaft 2 ist folglich im Bereich des Streckabschnitts 23 mit radialen Ausbuchtungen W und radialen Einbuchtungen E versehen. Die Ausbuchtungen W und die Einbuchtungen E wechseln einander in axialer Richtung ab und bilden die besagte ziehharmonikaartige Gestaltung H.

Fig. 3 zeigt den Streckabschnitt 23a in einem nicht gestreckten Zustand. Fig. 4 zeigt den Streckabschnitt 23a bei einer entsprechenden Zugbelastung F, F'. Die ziehharmonikaartige Gestaltung H wird unter der Einwirkung der Zugkraft F (und der Reaktionskraft F') axial in die Länge gezogen/gestreckt.

Es versteht sich, dass die in den Fig. 3 und 4 gezeigte Anzahl, Formgebung und Dimensionierung der Ausbuchtungen W und der Einbuchtungen E als rein exemplarisch zu verstehen sind.

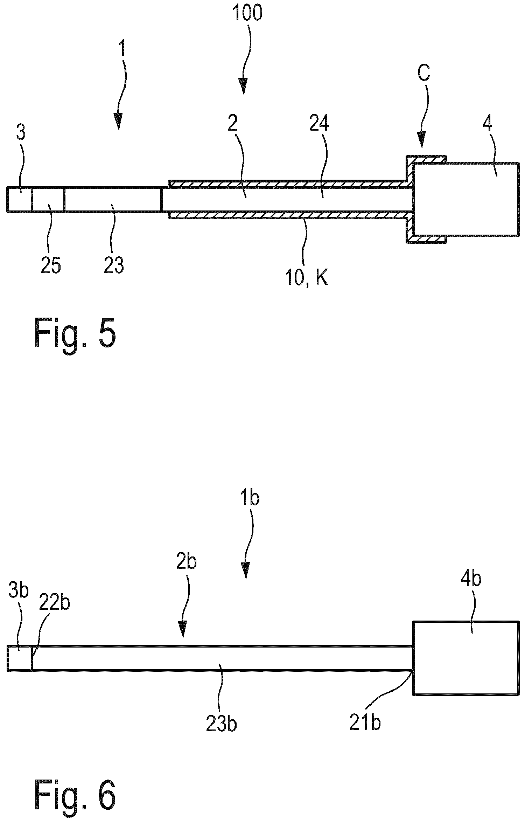

Fig. 5 zeigt eine Katheteranordnung 100 mit einem Katheter 1 nach den Fig. 1 und 2 und mit einer Einführhilfe 10. Die Einführhilfe 10 ist vorliegend als Kapillar K gestaltet. Die Einführhilfe 10 weist ein Lumen (ohne Bezugszeichen) auf, das zur Aufnahme des Katheterschafts 2 eingerichtet ist. In der in Fig. 5 gezeigten Konfiguration ist der Katheterschaft 2 über ein proximales Ende (ohne Bezugszeichen) der Einführhilfe 10 in das besagte Lumen eingeschoben, wobei das distale Ende des Katheterschafts 2 mitsamt der Katheterspitze 3 und dem Streckabschnitt 23 aus dem Lumen der Einführhilfe 10 distal hervorragt. Der Katheteransatz 4 und das proximale Ende der Einführhilfe sind zum Ausbilden einer lösbaren Verbindung C eingerichtet. Die lösbare Verbindung C kann eine Schraub-, Luer- oder sonstige für den vorliegenden Zweck geeignete Verbindung sein.

Die Fig. 6 bis 9 zeigen weitere Ausführungsformen erfindungsgemäßer Katheter 1b, 1c, 1d, 1e. Zur Vermeidung von Wiederholungen wird nachfolgend in erster Linie auf wesentliche Unterschiede der Katheter 1b, 1c, 1d, 1e gegenüber dem Katheter 1 und dem Katheter 1a nach den Fig. 1 bis 4 eingegangen. Funktionsgleiche Bauteile und/oder Abschnitte der Katheter 1b, 1c, 1d, 1e sind mit identischen Bezugszeichenziffern unter Hinzufügung von Kleinbuchstaben versehen. Sofern nichts anderes beschrieben ist, gilt das zu den Kathetern 1, 1a nach den Fig. 1 bis 4 Offenbarte, mutatis mutandis, auch für die Katheter 1b, 1c, 1d, 1e nach den Fig. 6 bis 9.

Bei dem Katheter 1b nach Fig. 6 ist der Streckabschnitt 23b über eine gesamte Länge des Katheterschafts 2b erstreckt. Hierdurch kann im Vergleich zu dem Katheter 1 nach den Fig. 1 und 2 eine einfachere Herstellung erreicht werden.

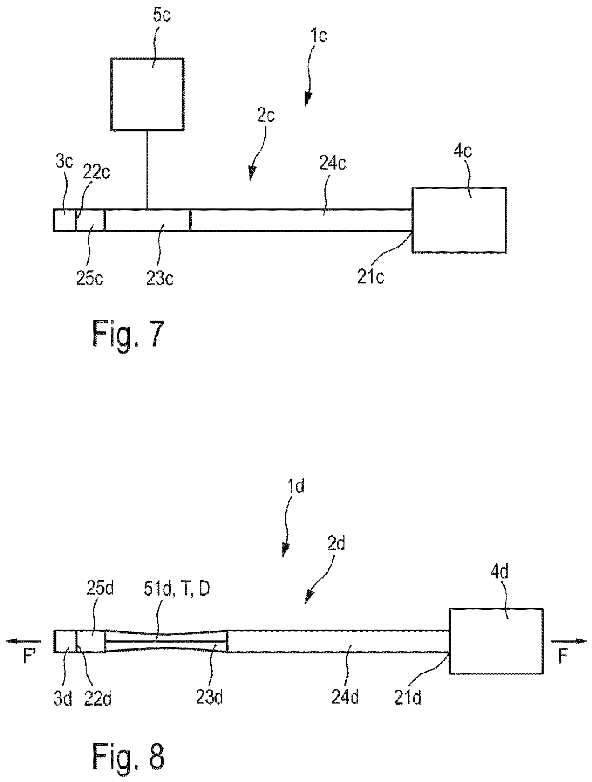

Der Katheter 1c nach Fig. 7 weist eine Streckungsbegrenzungseinrichtung 5c auf, die in Fig. 7 schematisch in Blockdarstellung gezeigt ist. Die Streckungsbegrenzungseinrichtung 5c ist dem Streckabschnitt 23c zugeordnet. Die Streckungsbegrenzungseinrichtung 5c ist zur Begrenzung der Streckung des Streckabschnitts 23c eingerichtet. Hierdurch kann einer übermäßigen Streckung und einer damit einhergehenden mechanischen Überbeanspruchung des Streckabschnitts 23c entgegengewirkt werden. Die Streckungsbegrenzungseinrichtung 5c ist bei unterschiedlichen Ausgestaltungen unterschiedlich gestaltet. Sobald der Streckabschnitt 23c eine maximal zulässige Streckung erreicht, wirkt die Streckungsbegrenzungseinrichtung vorzugsweise als Zugentlastung D und bewirkt eine Kraftübertragung zwischen dem proximalen Schaftabschnitt 24c und dem distalen Schaftabschnitt 25c unter Überbrückung des Streckabschnitts 23c.

Der Katheter 1c nach Fig. 8 zeigt eine Variante des Katheters 1c, wobei die Streckungsbegrenzungseinrichtung ein längserstrecktes Zugelement 51d aufweist. Das längserstreckte Zugelement überbrückt den (maximal zulässig gestreckten) Streckabschnitt 23d unter Ausbildung der besagten Zugentlastung D.

Bei der in Fig. 8 gezeigten Ausführungsform weist das längserstreckte Zugelement 51d einen leitfähigen Draht T auf, der die besagte Zugentlastung D ausbildet. Der leitfähige Draht T ist entlang und/oder in dem Streckabschnitt 23d längserstreckt und mechanisch mit dem proximalen Schaftabschnitt 24d und dem distalen Schaftabschnitt 25d verbunden. Alternativ oder zusätzlich kann der leitfähige Draht T mit dem Katheteransatz 4d und/oder der Katheterspitze 3d mechanisch verbunden sein. Dabei zeigt Fig. 8 lediglich einen Abschnitt des leitfähigen Drahts T.

Der Draht T ist vorliegend zum einen zum Ausbilden der besagten Zugentlastung D eingerichtet. Zum anderen ist der Draht T zur Übertragung und/oder Abgabe von Stromimpulsen zur Neurostimulation eingerichtet.

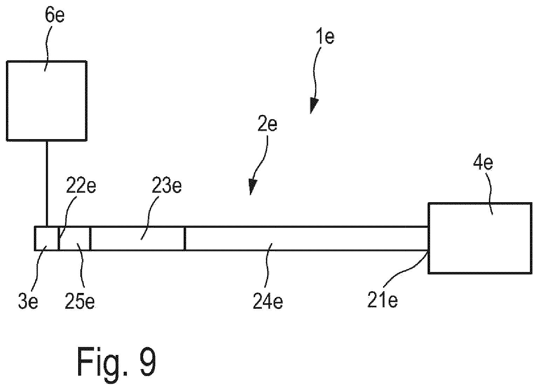

Der Katheter 1e nach Fig. 9 weist eine Fixiereinrichtung 6e auf, die schematisch in Blockdarstellung gezeigt ist. Die Fixiereinrichtung 6e ist der Katheterspitze 3e zugeordnet. Die Fixiereinrichtung 6e ist zum Fixieren der Katheterspitze 3e in proximaler Richtung eingerichtet, um eine proximale Dislokation der Katheterspitze 3e zu erschweren. Hierfür kann die Fixiereinrichtung 6e jede geeignete Form aufweisen. Beispielsweise kann die Fixiereinrichtung 6e an der Katheterspitze 3e ausgebildete oder angebrachte Fixierelemente aufweisen, die zur Interaktion mit einem die Katheterspitze 3e umgebenden Körpergewebe eingerichtet sind. Durch die besagte Interaktion kann die Reibung im Bereich der Katheterspitze 3e erhöht werden oder ein Formschluss mit dem Körpergewebe erreicht werden.

Es versteht sich, dass einzelne Merkmale der Katheter 1, 1a, 1b, 1c, 1d, 1e unter Ausbildung weiterer erfindungsgemäßer Ausführungsformen miteinander kombiniert werden können.

## Patentansprüche

1. Katheter (1, 1a bis 1e) zur Verabreichung eines Lokalanästhetikums, aufweisend
einen Katheterschaft (2, 2b bis 2e), der zwischen einem proximalen Ende (21, 21b bis 21e) und einem distalen Ende (22, 22b bis 22e) längserstreckt ist,
eine Katheterspitze (3, 3b bis 3e), die an dem distalen Ende (22, 22b bis 22e) angeordnet ist, und aufweisend
einen Katheteransatz (4, 4b bis 4e), der an dem proximalen Ende (21, 21b bis 21e) angeordnet ist,
**dadurch gekennzeichnet, dass** der Katheterschaft (2, 2a bis 2e) einen Streckabschnitt (23, 23a bis 23e) aufweist, der derart leicht streckbar ausgebildet ist, dass bei einer proximalen Zugbelastung (F) des Katheteransatzes (4, 4b bis 4e) eine wenigstens abschnittsweise Streckung des Katheterschafts (2, 2b bis 2e) erfolgt, wodurch eine proximale Dislokation der Katheterspitze (3, 3b bis 3e) vermieden ist.

2. Katheter (1, 1a bis 1e) nach Anspruch 1, **dadurch gekennzeichnet, dass** der Streckabschnitt (23, 23a bis 23e) eine Zugnachgiebigkeit (Z1) von wenigstens 10 mm/N, bevorzugt von wenigstens 100 mm/N, besonders bevorzugt von wenigstens 1000 mm/N aufweist.

3. Katheter (1a) nach Anspruch 1 oder 2, **dadurch gekennzeichnet, dass** der Streckabschnitt (23a) eine leicht streckbare Gestaltgebung (G) aufweist.

4. Katheter (1a) nach Anspruch 3, **dadurch gekennzeichnet, dass** die leicht streckbare Gestaltgebung (G) eine ziehharmonikaartige Gestaltgebung (H) ist.

5. Katheter (1, 1a bis 1e) nach einem der vorhergehenden Ansprüche, **dadurch gekennzeichnet, dass** der Streckabschnitt (23, 23a bis 23e) aus einem leicht streckbaren Material (M1) gefertigt ist.

6. Katheter (1, 1a bis 1e) nach Anspruch 5, **dadurch gekennzeichnet, dass** das leicht streckbare Material (M1) Polyethylen und/oder Silikon aufweist oder ist.

7. Katheter (1b) nach einem der vorhergehenden Ansprüche, **dadurch gekennzeichnet, dass** der Streckabschnitt (23b) über eine gesamte Länge des Katheterschafts (2b) längserstreckt ist.

8. Katheter (1, 1a, 1c bis 1e) nach einem der Ansprüche 1 bis 6, **dadurch gekennzeichnet, dass** der Streckabschnitt (23, 23a, 23c bis 23e) über lediglich einen Teil der Gesamtlänge des Katheterschafts (2, 2a, 2c bis 2e) längserstreckt ist und der Katheterschaft (2, 2a, 2c bis 2e) abseits des Streckabschnitts (23, 23a, 23c bis 23e) eine Zugnachgiebigkeit (Z2) aufweist, die maximal 20 %, bevorzugt maximal 5 %, besonders bevorzugt maximal 2 %, der Zugnachgiebigkeit (Z1) des Streckabschnitts (23, 23a, 23c bis 23e) beträgt.

9. Katheter (1, 1a, 1c bis 1e) nach Anspruch 8, **dadurch gekennzeichnet, dass** der Katheterschaft (2, 2a, 2c bis 2e) abseits des Streckabschnitts (23, 23a, 23c bis 23e) eine unterschiedliche Gestaltgebung aufweist und/oder aus einem unterschiedlichen Material (M2) gefertigt ist.

10. Katheter (1c, 1d) nach einem der vorhergehenden Ansprüche, **dadurch gekennzeichnet, dass** eine Streckungsbegrenzungseinrichtung (5c) vorhanden und zur Begrenzung der Streckung des Streckabschnitts (23c, 23d) eingerichtet ist.

11. Katheter (1d) nach Anspruch 10, **dadurch gekennzeichnet, dass** die Streckungsbegrenzungseinrichtung ein längserstrecktes Zugelement (51d) aufweist, das den Streckabschnitt (23d) unter Ausbildung einer Zugentlastung (D) überbrückt.

12. Katheter (1d) nach Anspruch 11, **dadurch gekennzeichnet, dass** das längserstreckte Zugelement (51d) einen elektrisch leitfähigen Draht (T) aufweist, der zur Übertragung und/oder Abgabe von Stromimpulsen zur Neurostimulation eingerichtet ist.

13. Katheter (1e) nach einem der vorhergehenden Ansprüche, **dadurch gekennzeichnet, dass** die Katheterspitze (3e) eine Fixiereinrichtung (6e) aufweist, die zum Fixieren der Katheterspitze (3e) in proximaler Richtung eingerichtet ist, um eine proximale Dislokation der Katheterspitze (3e) zu erschweren.

14. Katheteranordnung (100) mit einem Katheter (1, 1a bis 1e) nach einem der vorhergehenden Ansprüche und mit einer Einführhilfe (10), die zwischen einem distalen Ende und einem proximalen Ende längserstreckt ist und ein Lumen zum Einführen des Katheterschafts (2, 2a bis 2e) sowie einen an dem proximalen Ende angeordneten Konnektor zum Ausbilden einer lösbaren Verbindung (C) mit dem Katheteransatz (4, 4b bis 4e) aufweist.

## Claims

1. Catheter (1, 1a to 1e) for administering a local anaesthetic, comprising
a catheter shaft (2, 2b to 2e), which extends between a proximal end (21, 21b to 21e) and a distal end (22, 22b to 22e),
a catheter tip (3, 3b to 3e), which is arranged at the distal end (22, 22b to 22e), and
a catheter hub (4, 4b to 4e), which is arranged at the proximal end (21, 21b to 21e),
wherein the catheter shaft (2, 2a to 2e) has a stretch section (23, 23a to 23e) which is configured to be easily stretchable in such a way that, in the event of a proximal tensile loading (F) of the catheter hub (4, 4b to 4e), an at least partial stretching of the catheter shaft (2, 2b to 2e) takes place, as a result of which a proximal dislocation of the catheter tip (3, 3b to 3e) is avoided.

2. Catheter (1, 1a to 1e) according to Claim 1, wherein the stretch section (23, 23a to 23e) has a tensile compliance (Z1) of at least 10 mm/N, preferably of at least 100 mm/N, particularly preferably of at least 1000 mm/N.

3. Catheter (1a) according to Claim 1 or 2, wherein the stretch section (23a) has an easily stretchable design (G).

4. Catheter (1a) according to Claim 3, wherein the easily stretchable design (G) is an accordion-like design (H).

5. Catheter (1, 1a to 1e) according to any one of the preceding claims, wherein the stretch section (23, 23a to 23e) is made of an easily stretchable material (M1).

6. Catheter (1, 1a to 1e) according to Claim 5, wherein the easily stretchable material (M1) comprises or is polyethylene and/or silicone.

7. Catheter (1b) according to any one of the preceding claims, wherein the stretch section (23b) extends over an entire length of the catheter shaft (2b).

8. Catheter (1, 1a, 1c to 1e) according to any one of Claims 1 to 6, wherein the stretch section (23, 23a, 23c to 23e) extends over only a part of the total length of the catheter shaft (2, 2a, 2c to 2e), and the catheter shaft (2, 2a, 2c to 2e), away from the stretch section (23, 23a, 23c to 23e), has a tensile compliance (Z2) which is a maximum of 20%, preferably a maximum of 5%, particularly preferably a maximum of 2%, of the tensile compliance (Z1) of the stretch section (23, 23a, 23c to 23e).

9. Catheter (1, 1a, 1c to 1e) according to Claim 8, wherein the catheter shaft (2, 2a, 2c to 2e), away from the stretch section (23, 23a, 23c to 23e), has a different design and/or is made of a different material (M2).

10. Catheter (1c, 1d) according to any one of the preceding claims, further comprising a stretch-limiting device (5c) designed to limit the stretching of the stretch section (23c, 23d).

11. Catheter (1d) according to Claim 10, wherein the stretch-limiting device comprises an elongate tension element (51d) bridging the stretch section (23d) to form a strain relief (D).

12. Catheter (1d) according to Claim 11, wherein the elongate tension element (51d) comprises an electrically conductive wire (T) configured for the transmission and/or delivery of electrical current pulses for neurostimulation.

13. Catheter (1e) according to any one of the preceding claims, wherein the catheter tip (3e) comprises a fixing device (6e) configured for fixing the catheter tip (3e) in the proximal direction, in order to hinder a proximal dislocation of the catheter tip (3e).

14. Catheter assembly (100) comprising a catheter (1, 1a to 1e) according to any one of the preceding claims and comprising an insertion aid (10) extending between a distal end and a proximal end and having a lumen for insertion of the catheter shaft (2, 2a to 2e) and a connector, arranged at the proximal end, for forming a releasable connection (C) to the catheter hub (4, 4b to 4e).

## Revendications

1. Cathéter (1, 1a à 1e) pour l'administration d'un anesthésique local, comprenant
une tige de cathéter (2, 2b à 2e), qui s'étend longitudinalement entre une extrémité proximale (21, 21b à 21e) et une extrémité distale (22, 22b à 22e), et
une pointe de cathéter (3, 3b à 3e), qui est disposée au niveau de l'extrémité distale (22, 22b à 22e), et comprenant
un embout de cathéter (4, 4b à 4e), qui est disposé au niveau de l'extrémité proximale (21, 21b à 21e),
**caractérisé en ce que** la tige de cathéter (2, 2a à 2e) présente une section extensible (23, 23a à 23e), conçue de manière à être facilement extensible de telle sorte que, lors d'une sollicitation en traction proximale (F) de l'embout de cathéter (4, 4b à 4e), un allongement au moins sur une partie de la tige de cathéter (2, 2b à 2e) se produit, ce qui permet d'éviter une dislocation proximale de la pointe de cathéter (3, 3b à 3e).

2. Cathéter (1, 1a à 1e) selon la revendication 1, **caractérisé en ce que** la section extensible (23, 23a à 23e) présente une compliance à la traction (21) d'au moins 10 mm/N, de préférence d'au moins 100 mm/N, de manière particulièrement préférée d'au moins 1000 mm/N.

3. Cathéter (1a) selon la revendication 1 ou 2, **caractérisé en ce que** la section extensible (23a) présente une géométrie (G) facilement extensible.

4. Cathéter (1a) selon la revendication 3, **caractérisé en ce que** la géométrie (G) facilement extensible est une géométrie de type accordéon (H).

5. Cathéter (1, 1a à 1e) selon l'une des revendications précédentes, **caractérisé en ce que** la section extensible (23, 23a à 23e) est fabriquée à partir d'un matériau facilement extensible (M1).

6. Cathéter (1, 1a à 1e) selon la revendication 5, **caractérisé en ce que** le matériau facilement extensible (M1) comprend ou est du polyéthylène et/ou de la silicone.

7. Cathéter (1b) selon l'une des revendications précédentes, **caractérisé en ce que** la section extensible (23b) s'étend longitudinalement sur une longueur totale de la tige de cathéter (2b).

8. Cathéter (1, 1a, 1c à 1e) selon l'une des revendications 1 à 6, **caractérisé en ce que** la section extensible (23, 23a, 23c à 23e) s'étend longitudinalement sur seulement une partie de la longueur totale de la tige de cathéter (2, 2a, 2c à 2e) et **en ce que** la tige de cathéter (2, 2a, 2c à 2e) présente, en dehors de la section extensible (23, 23a, 23c à 23e), une compliance à la traction (Z2) qui représente au maximum 20 %, de préférence au maximum 5 %, de manière particulièrement préférée au maximum 2 %, de la compliance à la traction (Z1) de la section extensible (23, 23a, 23c à 23e).

9. Cathéter (1, 1a, 1c à 1e) selon la revendication 8, **caractérisé en ce que** la tige de cathéter (2, 2a, 2c à 2e) présente, en dehors de la section extensible (23, 23a, 23c à 23e), une géométrie différente et/ou est fabriquée à partir d'un matériau différent (M2).

10. Cathéter (1c, 1d) selon l'une des revendications précédentes, **caractérisé en ce qu'**un dispositif de limitation d'allongement (5c) est présent et est conçu pour limiter l'allongement de la section extensible (23c, 23d).

11. Cathéter (1d) selon la revendication 10, **caractérisé en ce que** le dispositif de limitation d'allongement présente un élément de traction allongé (51d), qui franchit la section extensible (23d) en formant une décharge de traction (D).

12. Cathéter (1d) selon la revendication 11, **caractérisé en ce que** l'élément de traction allongé (51d) comprend un fil électriquement conducteur (T), qui est conçu pour transmettre et/ou délivrer des impulsions de courant destinées à la neurostimulation.

13. Cathéter (1e) selon l'une des revendications précédentes, **caractérisé en ce que** la pointe de cathéter (3e) présente un dispositif de fixation (6e), qui est conçu pour fixer la pointe de cathéter (3e) dans la direction proximale afin de rendre plus difficile une dislocation proximale de la pointe de cathéter (3e).

14. Ensemble de cathéter (100) comprenant un cathéter (1, 1a à 1e) selon l'une des revendications précédentes et un dispositif d'introduction (10), qui s'étend longitudinalement entre une extrémité distale et une extrémité proximale et présente une lumière destinée à l'introduction de la tige de cathéter (2, 2a à 2e), ainsi qu'un connecteur disposé au niveau de l'extrémité proximale pour former une liaison amovible (C) avec l'embout de cathéter (4, 4b à 4e).
